Europäisches Patentamt

European Patent Office

Office européen des brevets

(11) Publication number: 0 349 965
A2

# (12) EUROPEAN PATENT APPLICATION

(21) Application number: 89112139.4

(22) Date of filing: 03.07.89

(51) Int. Cl.4: **C12N 15/00 , C12N 9/10 , C12P 9/00**

(30) Priority: 04.07.88 JP 164983/88

(43) Date of publication of application:
10.01.90 Bulletin 90/02

(84) Designated Contracting States:
**BE DE FR GB NL**

(71) Applicant: **MEIJI SEIKA KAISHA LTD.**
**4-16 Kyobashi 2-chome**
**Chuo-ku Tokyo(JP)**

(72) Inventor: **Murakami, Takeshi** c/o Meiji Seika
**Kaisha, Ltd**
**Pharmaceutical Res. Laboratories 760,**
**Morooka-cho**
**Kohoku-ku Yokohama-shi Kanagawa(JP)**
Inventor: **Anzai, Hiroyuki** c/o Meiji Seika
**Kaisha, Ltd**
**Pharmaceutical Res. Laboratories 760,**
**Morooka-cho**
**Kohoku-ku Yokohama-shi Kanagawa(JP)**
Inventor: **Kusama, Takeshi** c/o Meiji Seika
**Kaisha, Ltd**
**4-16 Kyobashi 2-chome Chuo-ku**
**Tokyo(JP)**
Inventor: **Satoh, Eriko** c/o Meiji Seika Kaisha,
**Ltd**
**Pharmaceutical Res. Laboratories 760,**
**Morooka-cho**
**Kohoku-ku Yokohama-shi Kanagawa(JP)**
Inventor: **Nagaoka, Kozo** c/o Meiji Seika
**Kaisha, Ltd**
**Pharmaceutical Res. Laboratories 760,**
**Morooka-cho**
**Kohoku-ku Yokohama-shi Kanagawa(JP)**

(74) Representative: **WILHELMS, KILIAN &**
**PARTNER Patentanwälte**
**Eduard-Schmid-Strasse 2**
**D-8000 München 90(DE)**

(54) Novel genes encoding transaminase.

(57) Transaminase genes derived from Streptomyces hydroscopicus, transaminases encoded by the transminase genes, plasmids having the transaminase genes inserted therein, actinomycetes trasformed with the plasmids and a method of producing L-phosphinothricin by amination of (3-carboxy-3-oxo-propyl)methylphosphinate using the transformants which enables enhanced productivity of L-phosphinothricin.

# NOVEL GENES ENCODING TRANSMINASE

## FIELD OF THE INVENTION

The present invention relates to transaminase genes, transaminases and a method of producing L-phosphinothricin using them.

## BACKGROUND OF THE INVENTION

The present inventors previously accomplished the invention that conversion of (3-carboxy-3-oxo-propyl)methylphosphinate (hereinafter referred to as OMPB) into L-phosphinothricin by utilizing transaminase of bacteria in the presence of amino group donors (EP-A-0249188: the disclosure of which is incorporated herein by reference). However, from the industrial viewpoint, the improved method capable of providing enhanced productivity of L-phosphinothricin has been desired.

## SUMMARY OF THE INVENTION

An object of the present invention is to provide a method of producing L-phosphinothricin by amination of OMPB economically, in a large quantity and in high yield.

The present inventors have found that two transaminases (herein referred to as AT-I and AT-II) produced by Streptomyces hygroscopicus SF-1293 NP50 are useful in the production of L-phosphinothricin from OMPB. The present inventors further found that the productivity of L-phosphinothricin can be enhanced by using actinomycetes which has inserted therein a plasmid carrying novel gene(s) encoding the above-described transaminase(s).

## BRIEF DESCRIPTION OF THE DRAWINGS

Fig. 1 shows tha amino acid sequence of N-terminal regions of AT-I and AT-II.

Fig. 2 shows the probe A and probe B.

Probe A: DNA sequence (synthetic probe) which corresponds to the amino acid sequence, the third to 20th amino acid from the N-terminus, of AT-I.

Probe B: DNA sequence (synthetic probe) which corresponds to the amino acid sequence, the first to 19th amino acid from the N-terminus, of AT-II.

Fig. 3 shows restriction enzyme maps of the AT-I and AT-II genes.

Fig. 4 shows DNA sequence corresponds to neighboring N-terminal regions of AT-I and AT-II. The underlined parts coincide with amino acid sequence determined by the Edman degradation.

Fig. 5 shows restriction enzyme maps of plasmids, pMSB515, pMSB526, and pMSB527. In the figure, ░░░░ shows AT-I gene-containing DNA fragment and ▉▉▉ shows AT-II gene-containing DNA fragment.

Fig. 6 shows protein analysis of Streptomyces hygroscipicus SF1293 NP50 with plasmids by means of SDS-polyacrylamide gel electrophoresis.

## DETAILED DESCRIPTION OF THE INVENTION

The isolation of two transminases, AT-I and AT-II, of Streptomyces hygroscopicus SF-1293 NP50 is performed as follows.

Streptomyces hygroscopicus SF-1293 NP50 has been deposited at the Fermentation Research Institute, Agency of Industrial Science and Technology of Japan under the accession number FERM BP-1368 in accordance with the Budapest Treaty.

2

This strain is cultivated in a medium containing the nutrients conventionally used for growth of actinomycetes. Examples of carbon sources include glucose, starch, glycerol, sucrose, maltose syrup and molasses. Examples of nitrogen sources include soybean meal, wheat germ, meat extract, peptone, dry yeast, corn steep liquer and ammonium sulfate. If required, inorganic salts such as calcium carbonate, sodium chloride, potassium chloride and phosphates may be added to the medium.

The cultivation of this strain is carried out under aerobic condition in liquid culture, preferably in submerged liquid culture, at a temperature of from 25 to 40°C for 1 to 4 days.

After cultivation, the culture is subjected to ultrasonication to obtain crude enzyme solution. This crude enzyme solution can be purified by ammonium sulfate fractionation and several column chromatography using synthetic adsorbents.

Analysis of the thus-obtained purified enzyme solution by SDS (sodium dodecyl sulfate)-polyacrylamide gel electrophoresis reveals that the molecular weight of two transaminases, namely AT-I and AT-II, is 42,000 daltons and 48,000 daltons, respectively.

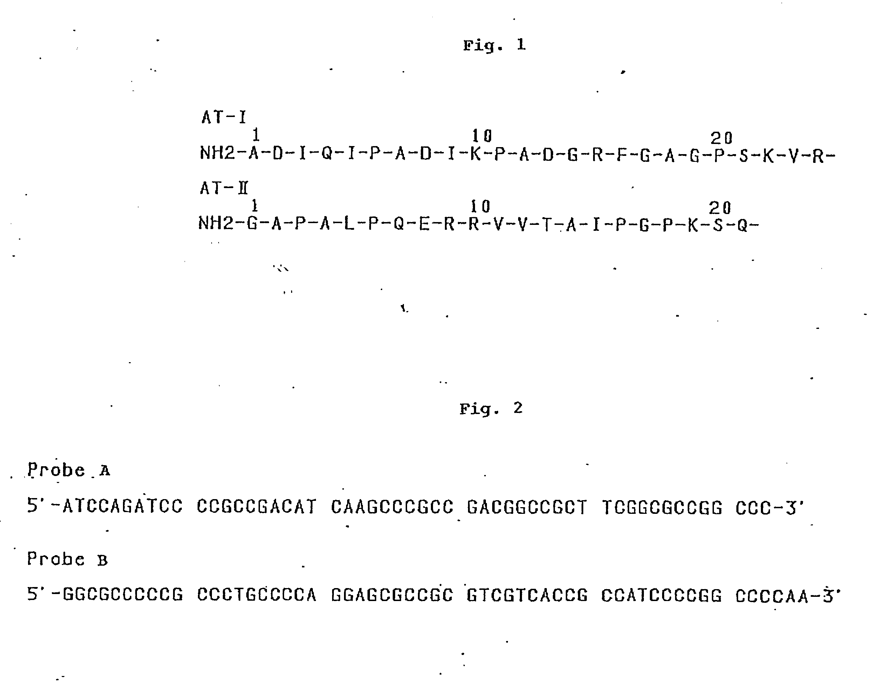

The amino acid sequences of N-terminal portion of AT-I and AT-II are analyzed by the Edman degradation. These N-terminal amino acid sequences are shown in Fig. 1.

Cloning of the transaminase genes according to the present invention is performed as follows.

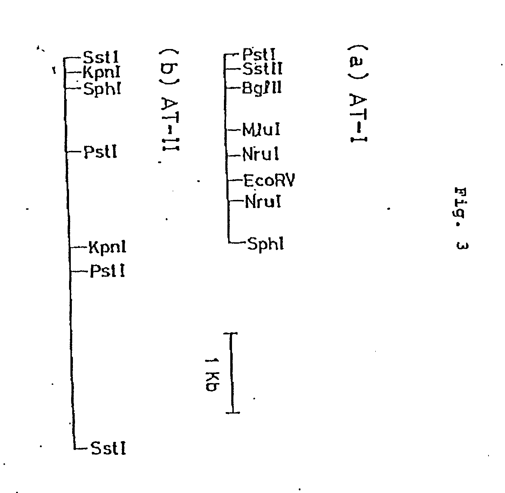

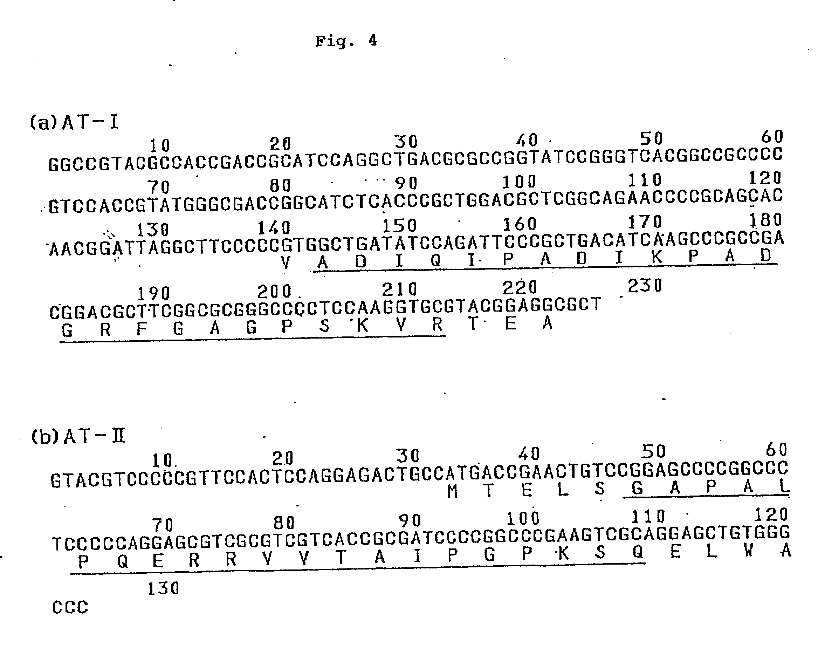

Two oligonucleotide probes corresponding to the N-terminal amino acid sequences of AT-I and AT-II are synthesized by DNA synthesizer (Fig. 2). Separately, the DNA library of the chromosomal DNA of Streptomyces hygroscopicus SF-1293 (FERM BP-130, ATCC 21705) is prepared in a conventional manner. Then, plaque hybridization is carried out using the two probes and clones which are hybridized with the probes are selected from the above-mentioned DNA library. Restriction enzyme maps of cloned genes for AT-I and AT-II are prepared (Fig. 3) and the base sequence of regions hybridized with the probes are determined by Southern hybridization. The base sequences determined by the dideoxy method (Fig. 4) are in agreement with those corresponding to the above-mentioned N-terminal amino acid sequences of AT-I and AT-II.

The recombinant plasmids according to the present invention can be prepared as follows.

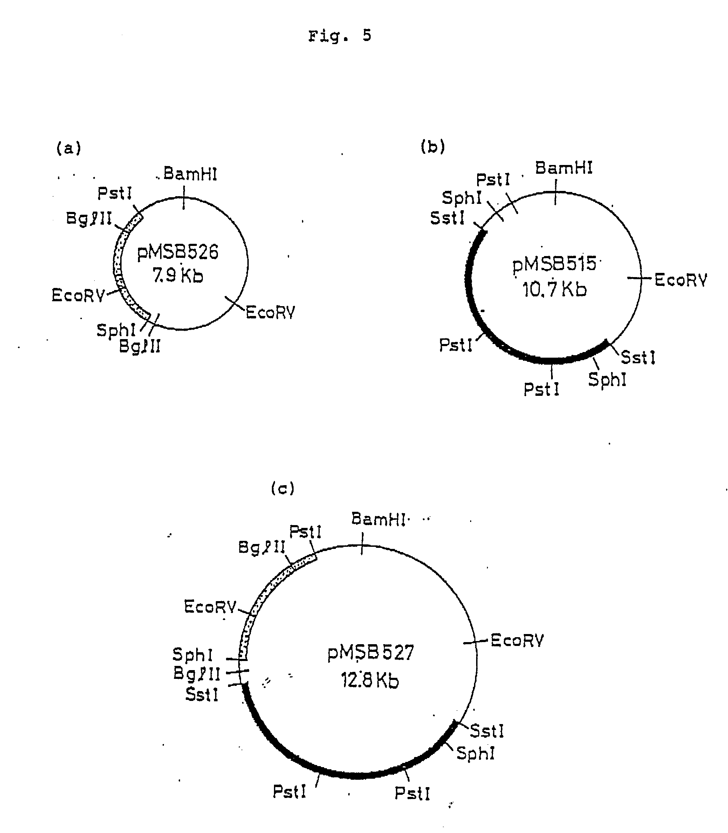

The AT-I gene-containing PstI-SphI DNA fragment (2.4 kb) is introduced into the actinomycetes vector plasmid pIJ702 (John Innes Culture Collection). Similarly, the AT-II gene-containing SstI-cleavage DNA fragment (5.0 kb) is introduced into the same vector plasmid. These recombinant plasmids are named as pMSB526 and pMSB515, respectively (Fig. 5). Further, SphI-BamHI large fragment of pMSB515 and SphI-BamHI small fragment of pMSB526 are ligated together to obtain the recombinant plasmid pMSB527 carrying both AT-I and AT-II genes (Fig. 5). Streptomyces lividans 66 (FERM BP-737) strains each harboring pMSB515, pMSB526 and pMSB527 have been deposited at the Fermentation Research Institute under the accession numbers FERM BP-2496, FERM BP-2497 and FERM BP-2498, respectively, in accordance with the Budapest Treaty.

Streptomyces hygroscopicus SF-1293 NP50 (FERM BP-1368) is transformed with each of the recombinant plasmids as obtained above. These transformants of the present invention can produce AT-I and AT-II in high yield.

The above-described gene manipulation procedures are performed in accordance with the conventional manner (cf. Genetic Manipulation of Streptomyces, A Laboratory-Manual, D.A. Hopwood et al, The John Innes Foundation, 1985).

The producing L-phosphinothricin according to the present invention is carried out as follows.

The transformants as obtained above are cultivated in the manner suitable for growth of host cells and the crude enzyme solution is recovered. OMPB and the amino group donors are added to the enzyme solution and the enzyme reaction is carried out.

As the enzyme solution, the culture of the transformants per se and the purified enzyme solution can be used.

OMPB and the amino group donor are added to the enzyme solution to conduct the enzyme reaction.

OMPB is a known compound as described in U.S. Patent 4,399,287.

Specific examples of the amino group donors include amino acids such as L-glutamic acid, L-aspartic acid, L-alanine, L-methionine, L-glutamine, L-leucine, L-isoleucine, L-valine, L-lysine, L-ornithine, L-histidine, L-phenylalanine, L-tyrosine, L-triptophane and alkali metal salts thereof. D-amino acids corresponding to these L-amino acids can also be used. The amino group donor is used alone or in combination of two or more. The combination of glutamic acid or salts thereof and aspartic acid or salts thereof is preferably used.

The final concentration of OMPB and the amino group donors are from 10 to 100 mg/ml, preferably from 30 to 50 mg/ml.

The pH value of the reaction mixutre is adjusted to pH 7.5 or more, preferably 8.0 to 9.0. The enzyme reaction is carried out at a temperature of 20 to 50°C for 1 to 48 hours, preferably 30 to 40°C for 8 to 24

3

hours.

L-phosphinothricin thus produced can be recovered from the reaction mixture in accordance with the method described in JP-A-57-47485. Namely, the reaction mixture is applied to the column in which cation exchange resin such as Dowex 50 W (Rohm and Haas) is packed so that the desired product which is adsorbed to the resin is eluted with water or diluted ammonia water.

It is well known that enzyme immobilized with organic solvents, cross linking agents, carriers and the like show the enhanced stability of the enzyme and it can be easily manipulated. The transminases, AT-I and AT-II, according to the present invention can be used in the immobilized form.

The following examples further illustrate the present invention in detail, but are not to be construed to limit the scope thereof.


EXAMPLE 1


(A) Purification of transaminases, AT-I, and AT-II

Streptomyces hygroscopicus SF-1293 NP50 was inoculated to the seed culture medium (containing 2.0% soluble starch, 1.0% polypeptone, 0.3% meat extract and 0.05% $K_2HPO_4$: pH = 7.0) and was incubated at 28°C for 24 hours with agitation. A portion of this seed culture was inoculated to the medium (containing 7.0% glucose, 3.9% wheat germ, 2.5% soluble vegetable protein, 0.3% $K_3PO_4$ and 0.0001% cobalt chloride: pH = 6.8) in an amount of 2 % based on the whole volume of the medium, and was incubated at 28°C for four days with agitation and aeration.

Three liter of the culture thus incubated was ultrasonicated and the resulting crude extract was subjected to ammonium sulfate fractionation and column chromatography using FPLC (Pharmacia). The columns and eluting buffers employed were as follows.

```
Step 1: column: Q-Sepharose (Pharmacia)
        resin volume : 100 ml.
        buffer: 100 mM Tris-HCl (pH 7.0)
        NaCl 0-1 M gradient.
Step 2: column: Mono Q (Pharmacia)
        buffer: 100 mM Tris-HCl (pH 7.0)
        NaCl 0-1 M gradient.
Step 3: column: Superose 12 (Pharmacia)
        buffer: 100 mM Tris-HCl (pH 7.0)
        100 mM NaCl.
Step 4: column: Mono Q (Pharmacia)
        buffer: 100 mM Tris-HCl (pH 7.0)
        NaCl 0-1 M gradient.
Step 5: column: Phenyl-Superose (Pharmacia)
        buffer: 100 mM calcium phosphate (pH 7.0)
        ammonium sulfate 1.0-0 M gradient.
Step 6: column: ProRPC (Pharmacia)
        buffer: H₂O (containing 0.1% trifluoroacetic
        acid) - 80% acetonitrile (containing
        0.1 % triluoroacetic acid).
```

In Step I, two fractions showing transaminase activity were obtained and the one fraction eluted at lower salt concentration was referred to as AT-I, and the another fraction eluted later as AT-II. The two fractions were subjected to the further processes individually, then 200 μg of AT-I and 250 μg of AT-II were obtained. As a result of 12.5% SDS-polyacrylamide gel electrophoresis, AT-I and AT-II were observed as a single band corresponding to a molecular weight of 42,000 daltons and 48,000 daltons, respectively.

(B) Analysis of N-terminal amino acid sequence

The Edman degradation of the purified AT-I and AT-II was carried out by a protein sequenser (Applied Biosystem model 470H) and the amino acid sequence of them from N-terminus were determined. For AT-I, up to the 24th amino acid from N-terminus, for AT-II, up to 21th were determined (Fig. 1).

(C) Synthesis of DNA probes

The DNA sequence which corresponds to amino acid sequence of from the third amino acid (isoleucine) to the 20th amino acid (proline) from N-terminus of AT-I, and that cooresponds to amino acid sequence of from N-terminal amino acid (glycine) to the 19th amino acid (lysine) of AT-II were determined.

The oligonucleotides, 53 bp (probe A) for AT-I and 56 bp (probe B) for AT-II, were synthesized by means of a DNA synthesizer (Applied Biosystem model 380A). The DNA sequences of them are shown in Fig. 2.

(D) Cloning of genes

Chromosomal DNA of Streptomyces hygroscopicus SF-1293 (FERM BP-130, ATCC 27105) was isolated by the method of Smith et al (Method in Enzymology 12, 545 (1967)) and partially digested with a restriction enzyme Sau3AI and the thus-obtained DNA fragment was ligated with lambda-phage vector EMBL3 (Strata Gene Inc.) previously cleaved with BamHI. This ligation product was introduced into Escherichia coli P2392 (Strata Gene Inc.) using in vitro packaging kit (Takara Shuzo). The transformants were incubated at 37°C for 16 hours of L-agar plate (containing 10 g Bacto-tryptone, 5 g Bacto-Yeast exatract, 10 g NaCl and 10g Bacto-agar, per liter) for plaque formation. Probe A (53 bp) and probe B (56 bp) prepared in the above (C) were labeled with $^{32}$P and plaques capable of hybridizing each probe were selected with plaque hybridization. Consequently, 16 plaques hybridized with probe A and 17 plaques hybridized with probe B were obtained from about 10,000 plaques. Considering the molecular weight of AT-I, a plaque which seemed to have a full-length DNA was selected out of 17 plaques hybridized with probe A. Similarly, a plaque out of 16 plaques hybridized with probe B was selected. According to the method of Grossburger et al. (Nucleic Acids Research, 15, (1987)), DNAs were prepared from the plaques which were hybridized with the synthetic probes. Restriction enzyme maps of each cloned DNAs were prepared and the Southern hybridization was performed using probe A and probe B labeled with $^{32}$P to thereby determine the regions hybridizing with the probes.

(E) Determination of DNA sequence

The DNAs thus obtained were cloned in M13 phage and base sequence of the regions which were hybridized with the probes were determined in accordance with the dideoxy method using a Sequenase® kit (USB Inc.). As a result, the presence of DNA sequence which corresponds to the N-terminal amino acid sequence of AT-I and AT-II as described in the above (B) was confirmed in the cloned genes for AT-I and AT-II.

(F) Subcloning of genes and their introduction into actinomycetes

The cloned gene for AT-I was digested with PstI and SphI and a DNA fragment of 2.4 kb was obtained. This fragment was ligated with actinomycetes vector pIJ702 (John Innes Culture Collection) which was previously digested with PstI and SphI. The thus-obtained recombinant plasmid designated as pMSB526 (Fig. 5(a)) was introduced into Streptomyces lividans 66 (FERM BP-737) according to the method as described in Genetic Manipulation of Streptomyces, A Laboratory-Manual, D.A. Hopwood et al., The John Innes Foundation, 1985. The resulting transformant has been deposited at the Fermentation Research Institute under the accession number FERM BP-2497 in accordance with the Budapest Treaty. Similarly, the cloned gene for AT-II was digested with SstI, the resulting 5.0 kb DNA fragment was ligated with pIJ702 previously digested with SstI and the recombinant plasmid pMSB515 (Fig. 5(b)) was obtained. Streptomyces lividans 66 harbouring pMSB515 has been deposited at the Fermentation Research Institute under the accession number FERM BP-2496 in accordance with the Budapest Treaty. Further, pMSB526 was digested with SphI and BamHI and separately pMSB515 was digested with SphI and BamHI and a large fragment of the former was ligated with a small fragment of the latter to obtain the recombinant plasmid pMSB527 which possessed both AT-I gene and AT-II gene. Streptomyces lividans 66 into which pMSB527 was introduced has been deposited at the Fermentation Research Institute under the accession number FERM BP-2498 in accordance with the Budapest Treaty.

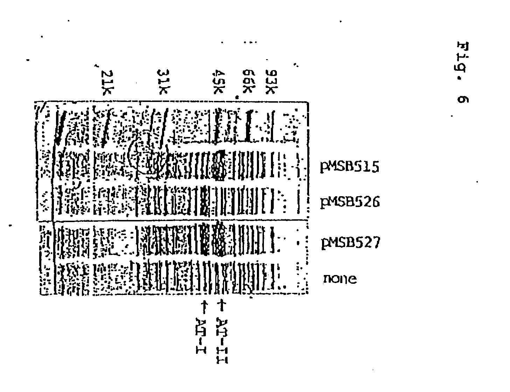

The thus-obtained recombinant plasmids, pMSB515, pMSB526 and pMSB527 were introduced into Streptomyces hygroscopicus SF-1293 NP50 (FERM BP-1368) and respective transformants were obtained. In the same manner as in the above (A), these transformants were cultured, and respective crude enzyme extracts were obtained. The analysis by SDS-polyacrylamide gel electrophoresis confirmed that AT-I and AT-II were produced in large quantity in the culture of the transformants (Fig. 6).

## EXAMPLE 2

Production of L-phosphinothricin using genetically engineered actinomycetes

Streptomyces hygroscopicus SF-1293 NP50 (FERM BP-1368) which possessed either pMSB515, pMSB526 or pMSB527 were inoculated to the seed culture medium as used in Example 1 (A) supplemented with 20 μg/ml thiostreptone and the control strain having no recombinant plasmid was also inoculated to the above-mentioned seed culture medium. Incubation was carried out at 28°C for 24 hours, and then a portion of each culture was inoculated in the total volume of the medium as used in Example 1 (A) in an amount of 2% based on the production medium and were incubated at 28°C for three days. A 1.25 ml- or 5 ml portion of the culture was taken out and (3-carboxy-3-oxo-propyl)methylphosphinate (OMPB), and sodium L-glutamate (L-Glu) and sodium L-asparate (L-Asp) as amino group donors were added thereto. Then 2.5 ml of 1 M Tris-HCl buffer (pH 8.5) was added to each culture solution to thereby adjust a pH of 8.5 and give a final volume of 25 ml. OMPB, L-Glu and L-Asp were each added so as to give the final concentration of 50 mg/ml. Thus, 20-fold and 5-fold diluted culture were prepared. The reaction was carried out at 37°C for 24 hours with gently shaken. Thereafter, the amount of L-phosphinothricin produced in the reaction mixture was analyzed with an amino acid analyzer (Atto MLC-703). The results were shown in Table 1.

Table 1

| Plasmid | culture/reaction mixture (v/v%) | Concentration of L-Phosphinotiricin (μg/ml) |
|---------|-------------------------------|---------------------------------------------|
| None | 20 | 33,000 |
| None | 5 | 16,000 |
| pMSB515 | 5 | 34,000 |
| pMSB526 | 5 | 35,000 |
| pMSB527 | 5 | 37,000 |

The 20-fold diluted culture of each transformant of the present invention showed productivity of L-phosphinothricin equivalent to or higher than that in case of using the 5-fold diluted control culture. In other words, the amount of L-phosphinothricin produced using the culture of the genetically engineered actinomycetes according to the present invention in spite of it was used in one fourth volume of the culture as obtained in the conventional method was comparable to that in the case of using the conventional method.

Thus, use of the actinomycetes into which the plasmid carrying transaminase (AT-I and AT-II) gene was introduced enables the reduction of the volume of the culture which is employed for conversion of OMPB into L-phosphinothricin.

Accordingly, it is expected that the cost required for the production of L-phosphinothricin which is useful as a herbicide can be reduced according to the present invention. Further, it is expected that L-phosphinothricin can be produced more economically, in larger amounts and in higher yield if the transformants and the enzymes of the present invention are used in the immobilized form.

While the invention has been described in detail and with reference to specific embodiments thereof, it will be apparent to one skilled in that the art that various changes and modifications can be made therein without departing from the spirit and scope thereof.

**Claims**

1. A gene coding for a protein having a molecular weight of 42,000 daltons and having transaminase activity, which is derived from a chromosomal DNA of Streptomyces hygroscopicus and has a restriction enzyme map as shown in Fig. 3(a).

2. A gene coding for a protein having a molecular weight of 48,000 daltons and having transaminase

7

activity, which is derived from a chromosomal DNA of Streptomyces hygroscopicus and has a restriction enzyme map as shown in Fig. 3(b).

3. A protein encoded by the gene of claim 1.

4. A protein encoded by the gene of claim 2.

5. A recombinant plasmid comprising the plasmid pIJ702 having an insert selected from the gene of claim 1, the gene of claim 2 and a fused gene comprising the gene of claim 1 and the gene of claim 2.

6. An actinomycetes containing therein the recombinant plasmid of claim 5.

7. A method of producing L-phosphinothricin by amination of (3-carboxy-3-oxo-propyl)-methylphosphinate which comprises using an actinomycetes of claim 6.

EP 0 349 965 A2

Fig. 1

AT-I
```
     1                           10                          20
NH2-A-D-I-Q-I-P-A-D-I-K-P-A-D-G-R-F-G-A-G-P-S-K-V-R-
```

AT-II
```
     1                           10                          20
NH2-G-A-P-A-L-P-Q-E-R-R-V-V-T-A-I-P-G-P-K-S-Q-
```

Fig. 2

Probe A

5'-ATCCAGATCC CCGCCGACAT CAAGCCCGCC GACGGCCGCT TCGGCGCCGG CCC-3'

Probe B

5'-GGCGCCCCCG CCCTGCCCCA GGAGCGCCGC GTCGTCACCG CCATCCCCGG CCCCAA-3'

Fig. 3

(a) AT-I

1 Kb

(b) AT-II

Fig. 4

(a) AT-I

GGCCGTACGCCACCGACCGCATCCAGGCTGACGCGCCGGTATCCGGGTCACGGCCGCCCC
GTCCACCGTATGGGCGACCGGCATCTCACCCGCTGGACGCTCGGCAGAACCCCGCAGCAC
AACGGATTAGGCTTCCCCCGTGGCTGATATCCAGATTCCCGCTGACATCAAGCCCGCCGA
                      V  A  D  I  Q  I  P  A  D  I  K  P  A  D

CGGACGCTTCGGCGCGGGCCCCTCCAAGGTGCGTACGGAGGCGCT
G  R  F  G  A  G  P  S  K  V  R  T  E  A

(b) AT-II

GTACGTCCCCCGTTCCACTCCAGGAGACTGCCATGACCGAACTGTCCGGAGCCCCGGCCC
                               M  T  E  L  S  G  A  P  A  L

TCCCCCAGGAGCGTCGGCGTCGTCACCGCGATCCCCGGCCCGAAGTCGCAGGAGCTGTGGG
P  Q  E  R  R  V  V  T  A  I  P  G  P  K  S  Q  E  L  W  A

CCC

EP 0 349 965 A2

Fig. 5

(a)

PstI
BgℓII
BamHI
pMSB526
7.9 Kb
EcoRV
SphI
BgℓII
EcoRV

(b)

PstI
SphI
SstI
BamHI
pMSB515
10.7 Kb
EcoRV
PstI
SstI
PstI
SphI

(c)

BgℓII
PstI
BamHI
EcoRV
SphI
BgℓII
SstI
pMSB527
12.8 Kb
EcoRV
SstI
SphI
PstI
PstI

Fig. 6